Europäisches Patentamt

(19) European Patent Office

Office européen des brevets

(11) Publication number: **0 145 176**
**B1**

(12) EUROPEAN PATENT SPECIFICATION

(45) Date of publication of patent specification: 08.06.88

(21) Application number: 84307113.5

(22) Date of filing: 17.10.84

(51) Int. Cl.⁴: **A 61 N 1/36,** A 61 H 39/00,
A 61 H 39/02

(54) Electro-therapeutic device.

(30) Priority: 25.10.83 JP 164090/83 u
16.11.83 JP 176085/83 u
27.06.84 JP 95399/84 u

(43) Date of publication of application:
19.06.85 Bulletin 85/25

(45) Publication of the grant of the patent:
08.06.88 Bulletin 88/23

(84) Designated Contracting States:
AT BE CH DE FR GB IT LI NL SE

(56) References cited:
AT-B- 351 668
DE-A-2 443 913
DE-A-2 638 580
DE-A-2 820 908
DE-A-2 928 826
DE-A-3 048 358
DE-A-3 202 010
FR-A-2 473 882
FR-A-2 500 745
FR-A-2 514 257
GB-A-1 416 141

(73) Proprietor: WACO CORPORATION OVERSEAS
LIMITED
4310 China Resources Building 26 Harbour Road
Hong Kong (HK)
(73) Proprietor: IRF YUGENGAISHA
15-15, Sekimachiminami, 2-chome
Nerima-ku Tokyo (JP)

(72) Inventor: Kairis, Alexandre Vladimirovich
15-15 Sekimuchiminami 1-chome Nerimaku
Tokyo (JP)

(74) Representative: Miller, Joseph et al
J. MILLER & CO. Lincoln House 296-302 High
Holborn
London WC1V 7JH (GB)

(56) References cited:
PROSPECTUS OF KRIEGER ELEKTRONIK, A-
1060 Vienna, Webgasse 11, Austria, delivered
at the Vienna International Trade Fair in the
pavillon of the Inventors' exibition in Sep.
1982, "Acupress"

Courier Press, Leamington Spa, England.

## Description

This invention relates to electro-therapeutic devices, such as are capable of detecting electrically low-resistance points such as acupuncture points or biologically active trigger points by applying the device to the surface of the skin and capable of stimulating them for a short period.

It is well-known that an appropriate stimulation on low-resistance points of the body can give a relief from pain, relaxation or health promotion. In the prior art, many electro-therapeutic devices are known which work efficiently for the low-resistance point stimulation, but conventional devices are relatively expensive, complex, bulky and generally require a trained person to operate. Moreover, the points of the body to be stimulated by such devices are very difficult for an untrained person to locate, thereby requiring a trained person or at least causing an untrained person to make a rough approximation.

Another disadvantage of such prior art devices, exemplified by FR—A—2,473,882, is that they require two separated electrodes. One of the electrodes must be held by the person to be treated and the second electrode is held and manipulated by the operator of the machine. It can be appreciated that when the person to be treated operates the prior art devices himself it presents an awkward or clumsy situation. Prior art devices in this category use separate electrodes which cause electrical current to travel through the body from one electrode to the other, thereby stimulating hundreds of low-resistance points and passing through vital organ's tissues unnecessarily.

GB—A—1,416,141 discloses an electro-therapeutic device for detecting the location of skin having an electrical resistance lower than the resistance of the surrounding skin and applying a stimulus thereto. The device comprises an electrical probe or electrode projecting from an insulating nose part in one end of a housing. The housing is electrically conductive and is held in the hand of the operator. Within the housing is an electronic assembly energisable from an internal battery through an external switch, which assembly applies a voltage between the housing and the probe or electrode. Should the resultant current exceed a predetermined value, due to a decrease in the electrical resistance of the skin to which the electrode is applied, an electric bulb is illuminated, indicating that a location of relatively low electrical resistance has been detected. Increased pressure upon the probe causes a larger stimulating current to flow. In the prior art device, the resistance detecting and stimulating currents pass through the body of the patient to whose skin the device is applied, and are not restricted to the locations to which the electrodes are applied.

Accordingly, there is a need for a simple portable electro-therapeutic device wshich can overcome these disadvantages, detect low-resistance points on the body, be easily efficiently operated by the user, and limit itself to stimulation of only those points required for treatment.

It is therefore an object of the present invention to provide an easy to use portable electro-therapeutic device that allows the user of the device to treat himself.

In accordance with the present invention, there is provided an electro-therapeutic device for detecting the location of skin having an electrical resistance lower than the resistance of the surrounding skin and applying a stimulus thereto, comprising a first electrode and a second electrode, the electrodes being electrically separated but mounted in close proximity in a housing and exposed so that both electrodes can be placed in contact with the skin, means coupled to the electrodes to detect an electrical resistance decrease of the skin between the electrodes; and means coupled to the electrodes for applying a stimulus to the skin having a relatively low resistance, such that the stimulus is limited to the area between the electrodes, when and only when the electrical resistance decrease of the skin between the electrodes is substantially equal to or greater than a predetermined value.

The objects and features of the present invention will become more apparent from a consideration of the following detailed description taken in conjunction with the accompanying drawings, in which:—

Figure 1 is a partially broken away front view of the device of the present invention;

Figure 2 is a schematic block diagram of the electric circuit for low-resistance point detection and stimulation;

Figure 3 is a cross-sectional view of the electrodes;

Figure 4 is a diagram of the electric circuit for generating a stimulating pulse;

Figure 5 is a schematic block diagram showing another embodiment of the electric circuit for low-resistance point detection and stimulation;

Figures 6 and 7 are respectively a perspective view and a bottom view showing another embodiment of the present invention;

Figure 8 is a schematic diagram showing another embodiment of the electric circuit for low-resistance point detection and stimulation;

Figure 9 is a cross-sectional view of another embodiment of electrodes whose separation distance can be adjusted;

Figures 10 to 14 are cross-sectional views each showing another embodiment of electrodes for low-resistance point detection and stimulation;

Figure 15 is a diagram of the electric circuit used in another embodiment of the device shown in Figures 16 to 19,

Figures 16 to 19 are respectively a front view, a side view, a top view and a bottom view showing another embodiment of the device of the present invention; and

Figure 20 is a cross-sectional view taken along the V—V line in Figure 17.

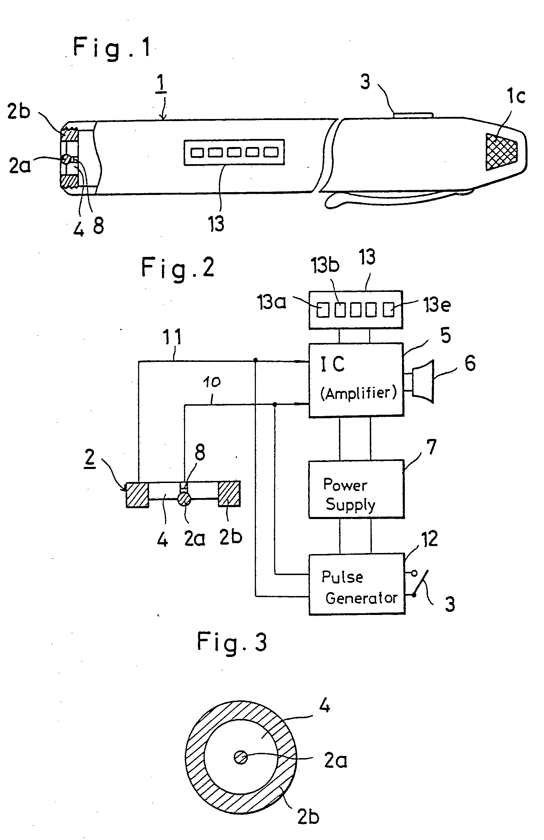

A first preferred embodiment (Figures 1 and 2) of an electro-therapeutic device according to the invention includes a housing 1 which is shaped in the form of a slender pen and has at its one end a low-resistance point sensor including a centre electrode 2a and an outer electrode 2b. The outer electrode 2b is disposed around the centre electrode 2a and spaced therefrom by insulative material 4. Each electrode is composed of a metal such as stainless steel and is 2 to 6 mm in diameter. These two electrodes 2a, 2b are disposed so closely as to be arranged in one unit.

The centre electrode 2a is connected through a spring contact 8 and a lead wire 10 to an IC circuit 5 including a DC amplifier, while the outer electrode 2b is connected to the same circuit 5 through a lead wire 11. The IC circuit 5 is powered by a battery 7 and its output is applied to a speaker 6 (or a lamp). The IC circuit 5 is further connected to an indicator 13 including light emitting diodes (LED's) 13a to 13e. For example, the indicator 13 is constructed to indicate a resistance between the electrodes 2a and 2b, so that LED's are turned on successively in response to a decrease of the resistace. Alternatively, all the LED's can first be turned on and turned off successively one by one with each stimulation on a low-resistance point.

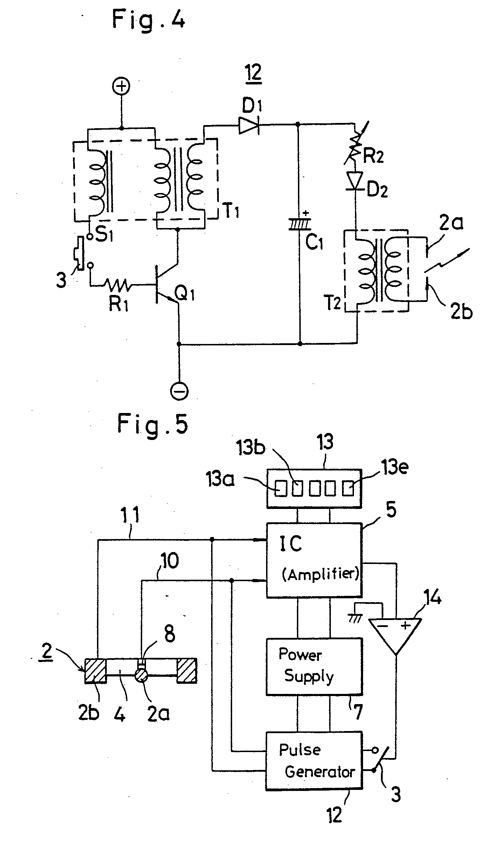

The electrodes 2a, 2b are connected to a pulse generator 12 which produces a pulse in response to an operation of a switch 3 disposed on the outside of the housing 1. The pulse generator 12 includes two transformers $T_1$, $T_2$, a transistor $Q_1$, a capacitor $C_1$ and two diodes $D_1$, $D_2$ as shown in Fig. 4.

All the components including the IC circuit 5, the battery 7, the speaker 6 and the pulse generator 12 are accommodated within the housing 1, but the sound emitting portion 1c of the speaker 6 appears on the surface of the housing 1.

In operation, an operator searches for a low-resistance point by holding the housing 1 with the sensor 2 placed in contact with his or his patient's skin. A current of approximately 0.2 micro A then flows through the electrodes 2a, 2b. When the electrodes 2a and 2b are located just upon a low-resistance point, the resistance between the two electrodes decreases, for instance, from 100 M ohm to 100 K ohm. This decrease in resistance activates the IC circuit 5 which drives the speaker 6 and indicator 13 so that the detection of the point may be audiovisually indicated. The operator then operates the switch 3 for stimulation.

The operating of the switch 3 causes the transistor $Q_1$ to be turned on, so that a current flows through the primary winding of the transformer $T_1$, and a boosted current flows through the diodes $D_1$, $D_2$ and the capacitor $C_1$ connected to the secondary winding thereof. The diodes $D_1$ and $D_2$ are made conductive or non-conductive in dependance upon the charge on the capacitor, thereby generating a pulse with a predetermined duty factor in the secondary winding. Since the secondary winding of the transformer $T_1$ is connected to the electrodes 2a, 2b, the pulse is applied to the low-resistance point to stimulate it.

The stimulation can be adjusted by variable resistor $R_1$ which varies the pulse frequency, that is, the output energy for stimulation.

Thus, the operation of the switch 3 at the time of detection of a low-resistance point causes an electric pulse (piezo-current about 1 micro A) to be generated, which flows through the detected low-resistance point to give a comfortable stimulation on the low-resistance point.

The switch 3 can be automatically turned on to apply the voltage through the electrodes when a low-resistance point is located.

Fig. 5 shows a preferred embodiment of this automatic detecting and stimulating operation. In Fig. 5, if a low-resistance point is detected, the detection signal is amplified through the IC circuit 5 to activate a comparator 14 with the detection displayed by the indicator 13. This causes the switch 3 to be operated to generate the pulse for stimulation by the circuit as shown in Fig. 4.

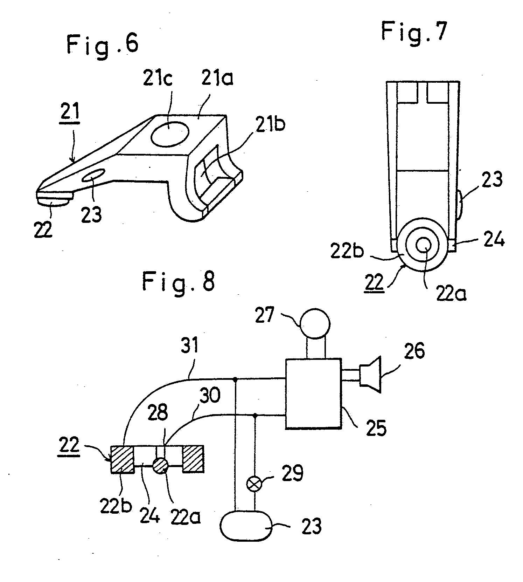

Figs. 6 and 7 show another preferred embodiment of the device in accordance with this invention. The electrotherapeutic device in Fig. 6 includes a housing 21 which is shaped to be able to be attached to a user's finger by having the finger inserted through an opening 21b at its front end, so that the user can make the the detection with ease. The housing is provided at the top end with a low-resistance point sensor 2 including a center electrode 22a and an outer electrode 22b disposed around the electrode 22a with both the electrodes separated by an insulative material 24. Similarly as mentioned before, each electrode is made of a metal such as stainless steel 2 to 6 mm in diameter. These two electrodes 22a, 22b are disposed very closely to form one unit, but preferably spaced away from each other at a distance shorter than at least about 3 cm.

The center electrode 22a is connected through a spring contact 8 and a lead wire 30 to an IC circuit 25, while the outer electrode 22b is connected to the same circuit 25 through a lead wire 31. The IC circuit 25 is powered by a battery 27 and its output is connected to a speaker 26 (and/or a lamp). The electrodes are also connected parallel to a series connection of a piezoelectric element 23 and a lamp 29 such as LED. All the components including the IC circuit 25, the battery 27 and the speaker 26 are accommodated within the surface 21a of the housing 21, but the sound emitting portion 21c of the speaker appears on the surface thereof.

In operation, the user searches for a low-resistance point by inserting one of his fingers into the opening 21b with the sensor 2 placed in contact with his or his patient's skin. A current of approximately 0.2 micro A flows through the electrodes 22a, 22b to the skin. If the electrodes 22a and 22b are located just upon a low-resistance point, the resistance between the electrodes decreases, for instance, from 100 M ohm to 100 K ohm. This decrease of resistance actuates the IC circuit 25, which then powers the speaker 26 to indicate the detection for the low-resistance point. The operater then actuates the piezoelectric element 23 mounted on the side of the housing 21 to make a stimulation. The actuation of the piezo-electric element causes the generation of a stimulus in the form of an electric pulse (piezzo current of approximately 1 micro A), which flows through the electrodes 22a, 22b into the detected low-resistance point to give a comfortable stimulation thereon. At the same time, the LED 29 is turned on for indication.

The speaker 26 could be replaced by an optical element such as an LED to visualize the low-resistance point detection. It can also be possible to drive the piezo-electric element automatically to apply a voltage between the electrodes upon the detection as mentioned above. Further, the electrodes can be driven by another power source instead of the piezo-electric element.

The detection of the low-resistance point is made using very low (0.2 micro Amp.) current and will cause hardly any pain or unpleasantness to the patient. Both the detection and stimulation are performed by the same electrodes. This allows very efficient and effective treatment. The use of the piezo-electric element as a stimulating power supply makes it possible to make a comfortable electric stimulation on the low-resistance points.

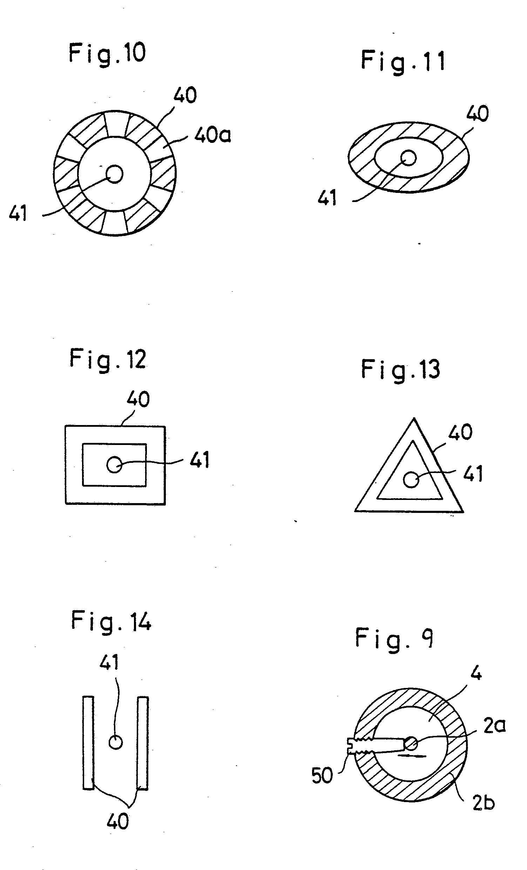

In the foregoing embodiments, the electrodes are shown to be coaxially and circularly arranged. However, the electrodes can be formed in any other shape. For example, an outer electrode 40 can be interrupted by gaps 40a arranged in equally spaced relationship as shown in Fig. 10. Alternatively, an oval, rectangular, or triangular electrode 40 can be provided as shown in Figs. 11 to 13. Fig. 14 shows another embodiment in which the electrodes are constructed as two parallel stripes 40, 41.

Furthermore, the electrodes 40, 41 do not always need to be exactly disposed in coaxial arrangement. The centre or outer electrode can be disposed with a certain offset. Only the requirement is that the outer and centre electrodes are closely disposed at a distance spaced away preferably less than 1 to 3 cm.

In each embodiment, it can be appreciated that the electrodes can be adjusted in distance. As shown Fig. 9, an adjusting screw penetrating through the outer electrode 2b is provided which can reach the center electrode 2a, so that the distance between the electrodes 2a, 2b can be adjusted by adjusting the screw 50.

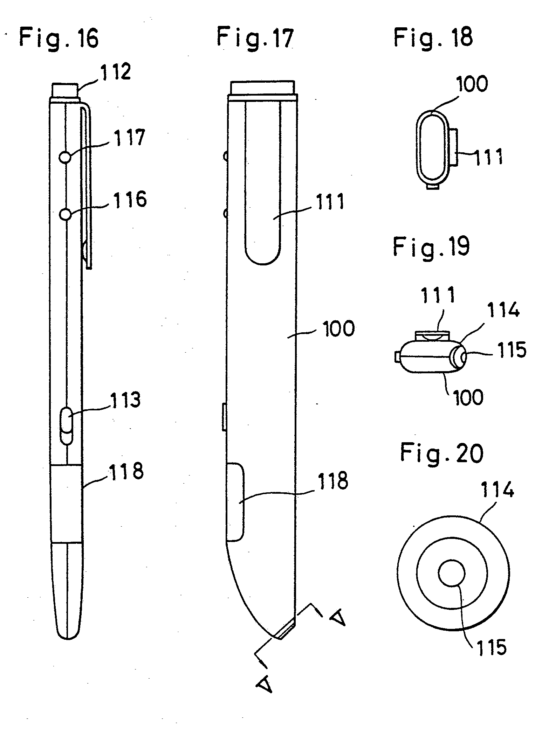

Referring now to Figs. 16 to 20, there is shown another embodiment of the present invention. The device in this embodiment includes a relatively slender housing 100 fabricated from plastic or other suitable material which is shaped to fit comfortably in the user's hand. A clip 111 is attached to the housing 100 to allow the electro-therapeutic device to be carried in a pocket. A pair of LED's 116, 117 and switches 112, 113, 118 which allow the user to operate the device are disposed in the housing 100. A pair of electrodes 114, 115 are disposed at the bottom of the housing 100.

Fig. 20 illustrates in cross section coaxial electrodes 114, 115. The coaxial electrodes are separated by an insulative material (including air) and are designed to be placed in contact with the user's skin. Preferably, the center electrode 115 is about 1 mm in diameter and the outer electrode 114 is about 4 mm in diameter.

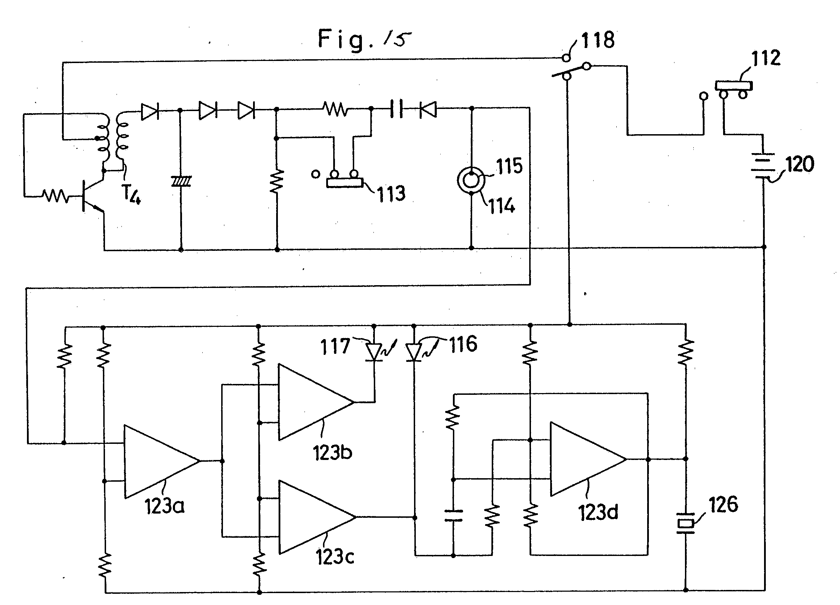

Fig. 15 shows a schematic diagram of an electronic circuit coupled to the coaxial electrodes 114, 115. The electronic circuit includes a 3V DC power supply 120, which is activated by a switch 112.

A DC 0.2 micro A current then flows to the electrodes 114, 115. When the electrodes 114, 115 are placed over a low-resistance point, there is a resistance differential or a decrease in resistance between the two electrodes which activates amplifier 123a. An activated output signal from the amplifier 123a, 123b is applied to illuminate a green LED 117, to sound a buzzer 126 and to operate a buzzer circuit which includes an amplifier 123d. When a low-resistance point is thus located, the switch 118 is depressed activating a pulse generator which includes transformers $T_4$. The pulse amplitude of the pulse generator can be changed by switch 113 to either a higher or a lower setting.

After stimulation of the low-resistance point, skin resistance changes. This change may be sensed by the amplifier 123c which in turn activates LED 116, and can provide an indication that the low-resistance point's resistance has been sufficiently chhanged to indicate that treatment is finished. This procedure may be repeated for other low-resistance points as desired.

Thus, it can be appreciated that a user of the present device merely has to hold the housing 100 and keep the electrodes 114, 115 in contact with a portion of a human body as he moves the device over the body. A very weak electric current (about 0.2 micro Amp.) flows over the human skin between the center electrode 115 and the outer electrode 114. When the electrodes 114, 115 come directly over a low-resistance point where electric resistance is much less than other areas (which varies between 100 M ohm and 100 K ohm), such variation in resistance is indicated by the sound of buzzer 126 and the LED 17. The user then pushes the switch 118 to send a pulse to the low-resistance point. The pulse is emitted between the electrodes 114, 115.

It can also be appreciated from the foregoing that the device of the present invention has several advantages over prior art devices. The entire instrument is small and lightweight and very easy to handle. The detection of a low-resistance point is made using very low (0.2 microamp.) current and will cause hardly any pain or unpleasantness to the patient. Both the detection and the stimulation are done by the same electrodes which allow very efficient and effective treatment. The degree of stimulation is adjustable in order to give the exact amount of required stimulation. Since both detection and stimulation are provided by the same electrodes, the construction of the instrument is very simple and also prevents the unnecessary flow of electric current to places other than the desired low-resistance points.

**Claims**

1. An electro-therapeutic device for detecting the location of skin having an electrical resistance lower than the resistance of the surrounding skin and applying a stimulus thereto, comprising a first electrode (2a; 22a; 41, 115) and a second electrode (2b; 22b, 40, 114), the electrodes being electrically separated but mounted in close proximity in a housing (1, 21, 100) and exposed so that both electrodes can be placed in contact with the skin, means (5, 25) coupled to the first and second electrodes to detect an electrical resistance decrease of the skin between the electrodes, and means (12, 23) coupled to the electrodes for applying a stimulus to the skin having a relatively low resistance, such that the stimulus is limited to the area between the electrodes, when and only when the electrical resistance decrease of the skin between the electrodes is substantially equal to or greater than a predetermined value.

2. A device according to claim 1, wherein the first electrode (2a, 22a, 41, 115) is surrounded by electrically insulative material (4, 24) which is in turn surrounded by the second electrode (2b, 22b, 40, 114).

3. A device according to claim 2, wherein the first and second electrodes (2a, 2b, 22a, 22b, 41, 40, 115, 114) are coaxially disposed in the housing (1, 21, 100).

4. A device according to claim 2, wherein the separation between the electrodes (2a, 2b) is adjustable (Figure 15).

5. A device according to claim 2, wherein the second electrode is circular (2b, 22b, 114), oval (Figure 11), rectangular (Figure 12) or triangular (Figure 13).

6. A device according to claim 2, wherein the second electrode (40) is interrupted by gaps (Figures 10 and 14).

7. A device according to any of claims 1 to 6, wherein the housing (100) is shaped in the form of a slender pen and provided with a clip (111) which allows the device to be carried in a pocket.

8. A device according to any of claims 1 to 6, wherein the housing (21) is shaped (21b) to be attached to the user's finger.

9. A device according to any of claims 1 to 8, wherein the means (5, 25) for detecting the electrical resistance decrease includes means (6, 13, 26, 117, 126) for providing the user of the device with an indication that the location has been detected.

10. A device according to claim 9, wherein the indication is provided by a light emitting diode (13, 17).

11. A device according to claim 9, wherein the indication is provided by a sound producing device (6, 26, 126).

12. A device according to any of claims 1 to 11, wherein the means for applying a stimulus includes a pulse generator (12, 23) for generating an electric potential.

13. A device according to claim 12, wherein said means for applying a stimulus includes a pulse generator comprised of transformers ($T_4$).

14. A device according to any of claims 1 to 13, wherein a stimulus is automatically applied to the skin when the decrease in resistance has been detected.

**Patentansprüche**

1. Elektrotherapeutische Vorrichtung, um die Lage von Hautstellen mit einem niedrigeren elektrischen Widerstand als die umgebende Haut aufzuspüren und diesen einen Reiz zu vermitteln, gekennzeichnet durch

eine erste Elektrode (2a, 22a, 41, 115) und eine zweite Elektrode (2b, 22b, 40, 114), wobei die Elektroden elektrisch getrennt, jedoch nahe benachbart in einem Gehäuse (1, 21, 100) angeordnet sind und freiliegen, so daß beide Elektroden (2a, 2b, 22a, 22b, 41, 40, 115, 114) in Kontakt mit der Haut gebracht werden können,

mit den ersten und zweiten Elektroden (2a, 2b, 22a, 22b, 41, 40, 115, 114) verbundene Mittel (5, 25), um einen elektrischen Widerstandsabfall der Haut zwischen den Elektroden aufzuspüren und

mit den Elektroden verbundene Mittel (12, 23), um der Haut mit relativ niedrigem Widerstand einen Reiz zu vermitteln, so daß der Reiz auf den Bereich zwischen den Elektroden beschränkt ist, wenn — und nur wenn — der elektrische Widerstandsabfall der Haut zwischen den Elektroden weitgehend einem vorgegebenen Wert entspricht oder größer ist.

2. Vorrichtung nach Anspruch 1, dadurch gekennzeichnet, daß die erste Elektrode (2a, 22a, 41, 115) durch elektrisch isolierendes Material (4, 24) umgeben ist, welches wiederum von der zweiten Elektrode (2b, 22b, 40, 114) umgeben ist.

3. Vorrichtung nach Anspruch 2, dadurch gekennzeichnet, daß die ersten und zweiten Elektroden (2a, 2b, 22a, 22b, 41, 40, 115, 114) in dem Gehäuse (1, 21, 100) koaxial angeordnet sind.

4. Vorrichtung nach Anspruch 2, dadurch gekennzeichnet, daß die Trennung zwischen den Elektroden (2a, 2b) einstellbar ausgebildet ist (Fig. 15).

5. Vorrichtung nach Anspruch 2, dadurch gekennzeichnet, daß die zweite Elektrode kreisrund (2b, 22b, 114), oval (Fig. 11), rechteckig (Fig. 12) oder dreieckig (Fig. 13) ausgebildet ist.

6. Vorrichtung nach Anspruch 2, dadurch gekennzeichnet, daß die zweite Elektrode (40) durch Spalten unterbrochen ist (Fig. 10 und 14).

7. Vorrichtung nach einem oder mehreren der Ansprüche 1 bis 6, dadurch gekennzeichnet, daß das Gehäuse (100) in der Form eines schlanken Stiftes geformt ist und mit einem Clip (111) versehen ist, welcher das Tragen der Vorrichtung in einer Tasche ermöglicht.

8. Vorrichtung nach einem oder mehreren der Ansprüche 1 bis 6, dadurch gekennzeichnet, daß das Gehäuse (21) eine dem Finger des Benutzers anliegende Formgebung (21b) aufweist.

9. Vorrichtung nach einem oder mehreren der Ansprüche 1 bis 8, dadurch gekennzeichnet, daß die Mittel (5, 25) zum Ermitteln des elektrischen Widerstandsabfalles Mittel (6, 13, 26, 117, 126) beinhalten, die dem Benutzer der Vorrichtung anzeigen, daß die Stelle aufgespürt wurde.

10. Vorrichtung nach Anspruch 9, dadurch gekennzeichnet, daß die Anzeige in Form einer LED (13, 117) ausgestaltet ist.

11. Vorrichtung nach Anspruch 9, dadurch gekennzeichnet, daß die Anzeige in Form einer tonerzeugenden Vorrichtung (6, 26, 126) ausgebildet ist.

12. Vorrichtung nach einem oder mehreren der Ansprüche 1 bis 11, dadurch gekennzeichnet, daß die Einrichtung zum Vermitteln eines Reizes einen Impulsgenerator (12, 23) zur Schaffung eines elektrischen Potentials beinhalten.

13. Vorrichtung nach Anspruch 12, dadurch gekennzeichnet, daß die Einrichtung zur Vermittlung eines Reizes einen aus Transformatoren (T4) bestehenden Impulsgenerator umfaßt.

14. Vorrichtung nach einem oder mehreren der Ansprüche 1 bis 13, dadurch gekennzeichnet, daß ein Reiz automatisch der Haut vermittelt wird, wenn ein Abfallen im Widerstand ermittelt wurde.

**Revendications**

1. Dispositif électrothérapeutique pour déceler l'emplacement de peau ayant une résistance électrique plus faible que celle de la peau environnante et lui appliquer un stimulus, caractérisé en ce qu'il comprend une première électrode (2a, 22a; 41, 115) et une seconde électrode (2b, 22b; 40, 114), les électrodes étant électriquement séparées mais montées en étroite proximité dans un logement (1, 21, 100) et exposées de manière à pouvoir être toutes deux mises en contact avec la peau, un moyen (5, 25) couplé aux première et seconde électrodes pour déceler une baisse de la résistance électrique présentée par la peau entre les électrodes, et un moyen (12, 23) relié aux électrodes pour appliquer un stimulus à la peau ayant une résistance relativement faible, de façon que le stimulus soit limité à la zone située entre les électrodes, quand et seulement quand la baisse de résistance électrique présentée par la peau entre les électrodes est sensiblement égale ou supérieure à une valeur déterminée.

2. Dispositif selon la revendication 1, caractérisé en ce que la première électrode (2a, 22a, 41, 115) est entourée par du matériau électriquement isolant (4, 24) lui-même entouré par la seconde électrode (2b, 22b, 40, 114).

3. Dispositif selon la revendication 2, caractérisé en ce que les première et seconde électrodes (2a, 2b, 22b, 41, 40, 115, 114) sont disposées coaxialement dans le logement (1, 21, 100).

4. Dispositif selon la revendication 2, caractérisé en ce que l'écartement entre les électrodes (2a, 2b) est réglable (figure 15).

5. Dispositif selon la revendication 2, caractérisé en ce que la seconde électrode est circulaire (2b, 22b, 114), ovale (figure 11), rectangulaire (figure 12) ou triangulaire (figure 13).

6. Dispositif selon la revendication 2, caractérisé en ce que la seconde électrode (40) est interrompue par des brèches (figures 10 et 14).

7. Dispositif selon l'une des revendications 1 à 6, caractérisé en ce que le logement (100) est conformé sous la forme d'un crayon mince et muni d'une attache (111) qui permet de porter le dispositif dans la poche.

8. Dispositif selon l'une des revendications 1 à 6, caractérisé en ce que le logement (21) est conformé pour être fixé au doigt de l'utilisateur.

9. Dispositif selon l'une des revendications 1 à 8; caractérisé en ce que le moyen (5, 25) pour déceler la baisse de résistance électrique comporte un moyen (6, 13, 26, 117, 126) pour fournir à l'utilisateur du dispositif une indication que l'emplacement a été décelé.

10. Dispositif selon la revendication 9, caractérisé en ce que l'indication est fournie par une diode électroluminescente (13, 17).

11. Dispositif selon la revendication 9, caractérisé en ce que l'indication est fournie par un dispositif générateur de son (6, 26, 126).

12. Dispositif selon l'une des revendications 1 à 11, caractérisé en ce que le moyen d'application d'un stimulus comporte un générateur d'impulsions (12, 23) pour la génération d'un potentiel électrique.

13. Dispositif selon la revendication 12, caractérisé en ce que ledit moyen d'application de stimulus comporte un générateur d'impulsions constitué par des transformateurs (T4).

14. Dispositif selon l'une des revendications 1 à 13, caractérisé en ce qu'un stimulus est automatiquement appliqué à la peau quand la baisse de résistance a été décelée.

Fig.1

Fig.2

Fig.3

## Fig.4

## Fig.5

Fig.6

21c  21a

21

21b

22  23

Fig.7

23

22b  24

22  22a

Fig.8

27

31  26

28  30

25

22

29

22b  24  22a

23

Fig.10

40
40a
41

Fig.11

40
41

Fig.12

40
41

Fig.13

40
41

Fig.14

41
40

Fig.9

4
2a
50
2b

4

Fig. 15

0 145 176

# Fig. 16

112
117
116
113
118

# Fig. 17

111
100
118

# Fig. 18

100
111

# Fig. 19

111
114
115
100

# Fig. 20

114
115